# EUROPEAN PATENT SPECIFICATION

(11) **EP 0 454 427 B1**
(45) Date of publication and mention of the grant of the patent: **06.12.1995**
(21) Application number: 91303664.6
(22) Date of filing: 24.04.1991
(51) Int. Cl.: C07F 9/547, A61K 31/675

(54) **Antiretroviral bases**
Antiretrovirale Basen
Bases anti-rétrovirales

(30) Priority: 24.04.1990 CS 2047/90
(43) Date of publication of application: 30.10.1991
(73) Proprietor: INSTITUTE OF ORGANIC CHEMISTRY AND BIOCHEMISTRY OF THE ACADEMY OF SCIENCES OF THE CZECH REPUBLIC, 166 10 Praha 6 (CZ); Rega Stichting, B-3000 Leuven (BE)
(72) Inventor: Holy, Antonin, Horni Porcenice, (CS); Jindrich, Jindrich, Praha 7, (CS); Balzarini, Jan, B-3030 Egenhoven (BE); De Clercq, Erik, B-3042 Bierbeek (BE)
(74) Representative: Lord, Hilton David

(56) References cited:
- EP-A- 0 253 412
- EP-A- 0 269 947
- EP-A- 0 270 885

## Description

The present invention relates to N-(3-substituted-2-phosphonylmethoxypropyl)purin-9-yl/pyrimidin-1-yl derivatives, methods of their preparation and their use as active components of antiviral preparations.

Particularly intractable virus-associated conditions are those caused by retroviruses, such as HIV, which induces AIDS. Curing these infections is very difficult or impossible and, although several compounds of widely varying structure, such as sulphated polysaccharides, aurinetricarboxylic acid, Evans Blue, glycorrhizine, heparin, and tetrahydroimidazo-[4,5,1-jk][1,4] benzodiazepine-2(1H)-thiones, show significant effects in vitro, only modified analogues of nucleosides and nucleotides, such as 2′,3′-dideoxycytidine, and 2′,3′-dideoxy-2′,3′-didehydrothymidine have been effective in the treatment of experimental animals. For AIDS, only 3′-azido-2′,3′-dideoxythymidine (AZT, zidovudine) and 2′,3′-dideoxyinosine (ddI) have so far been acknowledged as being effective.

Another important group of compounds that are, inter alia, active against retroviruses, is represented by the acyclic nucleotide analogues, for example, the 9-(2-phosphonylmethoxyethyl)purines (CZ-A-263951), of which the 9-(2-phosphonylmethoxyethyl)adenine was disclosed (CZ-A-263952). Tests with experimental animals have proven its effect on Moloney sarcoma virus, murine leukaemia virus, simian immunodeficiency virus (SIV), and in HIV tissue cultures (E. DeClercq, Antiviral Res., 12, 1, (1989)).

A particularly remarkable quality of antiviral compounds, especially the antiretroviral compounds, is the narrow structural efficacy margin of these compounds, activity being extremely sensitive to any structural modifications made in any substituents, such as in the 2-phosphonyl-methoxyethyl group of certain known compounds.

Introduction of alkyl groups, elongation or shortening of the chain, or removal of the oxygen atom leads invariably to loss of antiviral activity (A. Holy et al., Nucleotide Analogues as Antiviral Agents, ACS Symposium Series No 401, p51 (1989)).

The only substitution so far discovered which does not result in the loss of antiviral activity is the introduction of a hydroxymethyl group into the 2-position of the side-chain. However, this compound is active only against DNA viruses (HSV, adenoviruses, poxviruses, - CZ-A-233665 and CZ-A-264222 = EP-A-0253412), there being no detectable activity against the retroviruses. The corresponding aminomethyl, alkoxymethyl and azidomethyl derivatives show no antiviral activity whatsoever, the same being true for compounds in which the hydroxymethyl group is replaced by a group such as a methyl group (A. Holy et al., supra).

Particular examples of compounds which have been studied for their antiviral activity are the purin-9-yl and adenin-1-yl compounds substituted with 3-hydroxy-2-phosphonylmethoxypropyl groups or 2-(phosphonylmethoxy)ethyl groups. Any substituent other than a hydroxymethyl group invariably resulted in loss of all antiviral activity.

We have now found that substitution of the hydroxy group with fluorine both retains the antiviral activity of the compounds, while also serving to specifically target retroviruses and, furthermore, the antiretroviral activity does not correspond to the effects of the prior art compounds.

Accordingly, in a first aspect, the present invention provides compounds of formula (I) 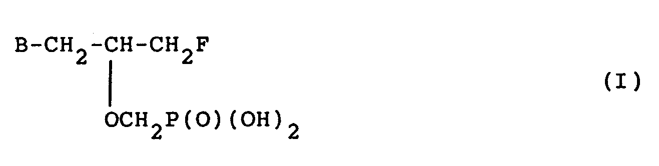
wherein B represents a purin-9-yl or pyrimidin-1-yl moiety, and pharmaceutically acceptable derivatives thereof, especially the alkali metal salts, ammonia or amine salts.

It will be appreciated that the configuration at the C-2 carbon atom may be S, R or RS.

The purin-9-yl and pyrimidin-1-yl groups of the invention are any such groups as are recognised in the art, such as xanthinyl, hypoxanthinyl and inosinyl groups. In particular, the groups are preferred to have the essential ring structure of either of the base types, either lacking all substituents, or having one or more alternative substituents, or a mixture of both, such as hypoxanthine.

Particularly suitable substituents on either of the ring types are such as amino groups, hydroxy groups, keto groups, cyano groups, alkylamino-groups, halogen atoms, thiol groups, lower alkyl groups, either unsubstituted or further substituted with similar groups as defined above, and alkenyl or alkynyl groups, unsubstituted or substituted as defined for alkyl groups. Other suitable groups are apparent to those skilled in the art.

Compared with the known compounds, the compounds of the invention have a better selectivity index (ratio of the mean minimum antiviral concentration to the mean cytotoxic concentration), which is a particular advantage of the present invention, and represents an important criterion in the development of potential antiviral drugs.

The invention further provides a method of producing compounds of formula (I), which comprises reacting compounds of formula (II) 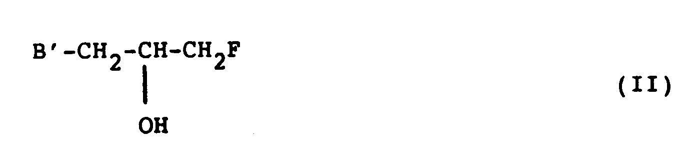
where B′ is a purin-9-yl or pyrimidin-1-yl moiety or its derivatives protected with O-alkyl, N-acyl or N-dialkylaminomethylene groups, and wherein the absolute configuration at the C-2 carbon atom may be S, R or RS, with dialkyl p-toluenesulfonyloxymethylphosphonates of the general formula III

4-CH₃C₆H₄SO₂OCH₂P(O)(OR¹)₂ (III)

wherein R¹ is an alkyl (C₁-C₃), preferably 2-propyl group, in the presence of 1 - 5 molar equivalents (relative to compounds of the general formula II) of sodium hydride in a dipolar aprotic solvent, preferably dimethylformamide at temperatures -30°C to 1OO°C, whereupon the solvent is evaporated and the protecting group on the heterocyclic base is removed by methanolysis or acid hydrolysis and, after isolation or deionisation at room temperature, the compounds of the general formula IV 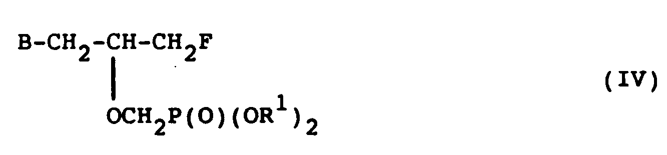
wherein B signifies the same as in the formula I and R¹ is the same as in formula III, are reacted with bromotrimethylsilane in an aprotic solvent, preferably in acetonitrile and, after removal of the solvent, the reaction mixture is treated with water or buffer solutions and the compounds of the general formula I are isolated, preferably by ion-exchange chromatography.

A method for the production of a preferred compound, wherein B is hypoxanthin-9-yl, comprises reaction of a compound of the formula I, where B is adenin-9-yl, with 3-methylbutyl nitrite, preferably in 50 - 80% aqueous acetic acid or with an aqueous solution of sodium nitrite in the presence of an equimolecular amount of acetic acid at room temperature.

An alternative method for the production of another preferred compound, wherein B is xanthin-9-yl, comprises reaction of a compound of formula I, wherein B is guanin-9-yl, with 3-methylbutyl nitrite, preferably in 50 - 80% aqueous acetic acid or with an aqueous solution of sodium nitrite in the presence of an equimolecular amount of acetic acid at room temperature.

Further, there is provided a method of producing a compound of formula I, wherein B is 2,6-diaminopurin-9-yl, comprising reaction of a compound of formula I, where B is 2-amino-6-azidopurin-9-yl, with gaseous hydrogen in the presence of a catalyst, preferably metallic palladium, prereduced on an inorganic carrier, in water or in dilute aqueous solutions of organic or mineral acids.

The present invention also provides use of the compounds of the invention in the manufacture of a medicament for the treatment or prophylaxis of a retroviral condition.

The invention also makes possible the utilisation of compounds of the general formula I for suppressing multiplication of viruses, particularly retroviruses, and application of these compounds to treatment of diseases caused by these viruses.

Useful pyrimidine bases in the general formula I include the compounds of the following formula (V), which include common natural bases such as uracil, thymine and cytosine: 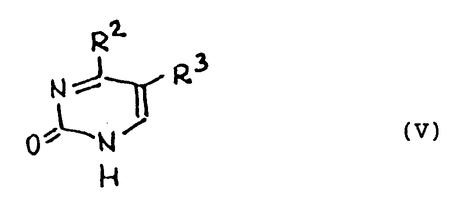
wherein R² is a hydrogen atom, a hydroxylamino, hydrazino, alkoxy, amino or substituted amino group and R³ is a hydrogen or halogen atom or an alkyl (C₁-C₃) group, or moieties of the general formula VI 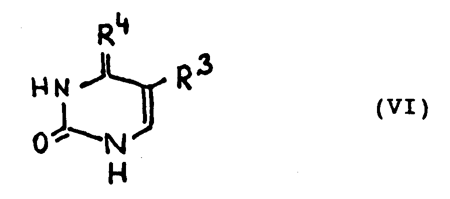
wherein R³ is as in formula V and R⁴ is an atom of oxygen or sulphur, as well as their synthetic analogues such as aza (5-aza, 6-aza) or deaza (3-deaza) derivatives.

Suitable purine bases include natural bases such as adenine, hypoxanthine, guanine, xanthine, and general moieties of formula VII 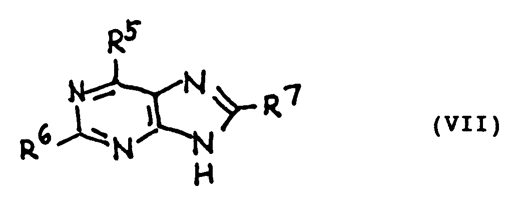
where R⁵ and R⁶ are a hydrogen or halogen atom, an amino, hydrazino, hydroxylamino, azido, substituted amino, hydroxy, alkoxy, mercapto, alkylmercapto group, or an alkyl group with a short carbon chain (C₁-C₄), and combination of these groups, and R₇ is an atom of hydrogen or halogen, a hydroxy, alkoxy, mercapto, alkylthio, amino or substituted amino group, and synthetic analogues, such as aza (2-aza, 8-aza) or deaza (1-deaza, 3-deaza or 7-deaza) derivatives.

Compounds of the general formula I advantageously can be prepared in accordance with the present invention, generally by the introduction of a phosphonomethyl group to the free hydroxy group of N-(3-fluoro-2-hydroxypropyl) derivatives of heterocyclic bases of the general formula II. These compounds are readily available, such as by alkylation of heterocyclic bases with fluoromethyloxirane (CZ-A-2048-90).

To exclude side-reactions during introduction of the organophosphorus moiety, those compounds of the formula II that contain a basic amino group, (e.g. cytosine, adenine and guanine) should be protected by introduction of a suitable protecting group, e.g. by aroylation (benzoylation), by transformation into the amidine functionality or otherwise (c.f. A. Holy, I. Rosenberg, H. Dvorakova: Collect. Czech. Chem. Commun. 54, 2470 (1989)), using, e.g. the specific N-benzoylation method described by G.S. Ti, B.L. Gaffney, and R.A. Jones (J. Am. Chem. Soc. 104, 1316 (1982)).

The prepared protected derivatives of formula II are then condensed with the reagent of formula III in the presence of sodium hydride, preferably in dimethylformamide. Best results are obtained if the reaction is performed with 3 - 3.5 molar equivalents of the hydride (relative to compound II). The alkylation reagent of the general formula III may be obtained according to the previously described method (A. Holy, I. Rosenberg: Collect. Czech. Chem. Commun. 47, 3447 (1982); A.F. Kluge: Org. Syn. 64, 80 (1985)); the ester group may be an alkyl or an aralkyl group. Since, however, under the given reaction conditions, compounds of the formula III may (similarly as neutral esters of phosphoric and phosphonic acids) behave as alkylation reagents, it is preferable to use esters with a low alkylation potency such as, e.g. the 2,2-dimethylpropyl or 2-propyl ester. The latter is easily preparable, e.g. by the procedure described in the accompanying Preparative Example.

The reaction of compounds of the formula III with anions of compounds of the formula II proceeds well at low temperatures (-1O°C to 0°C); it also may be performed at elevated temperatures. The reaction mixture is worked up preferably by removal of the protecting group (if used) from the heterocyclic base and isolation of the diester of the general formula IV by chromatography either on silica gel or an ion-exchanger. Under the deionisation conditions, the di(2-propyl)esters are stable even in dilute ammonia. After drying, compounds of the formula IV may be subjected to reaction with bromotrimethylsilane or iodotrimethylsilane in dimethylformamide, chlorinated hydrocarbons or preferably in acetonitrile. The reaction proceeds usually smoothly at room temperature.

After evaporation and codistillation of the residue with water, the crude compounds of the general formula I may be purified, preferably by ion-exchange chromatography and stored, preferably in the form of well water-soluble and stable alkali metal salts.

Compounds of the general formula I contain in the position 2 of the side-chain an asymmetric carbon atom. The described method of preparation is equally applicable to both enantiomers (R and S) as well as for the racemic derivative and depends on the character of the starting compound of general formula II.

Further preferred features of the invention are as follows.

A method of producing compounds of formula I, which consists in the reaction of N-(3-fluoro-2-hydroxypropyl) derivatives of pyrimidine and purine heterocyclic bases of formula II, as defined, where B′ is a purin-9-yl or pyrimidin-9-yl moiety or its derivatives protected with O-alkyl, N-acyl or N-dialkylaminomethylene group, and the absolute configuration at the C-2 atom is S, R or RS, with dialkyl p-toluenesulphonyloxymethylphosphonates of formula III, as defined, wherein R¹ is an alkyl (C₁-C₃), preferably 2-propyl, group, in the presence of 1 - 5 molar equivalents (relative to compounds of the general formula II) of sodium hydride in a dipolar aprotic solvent, preferably dimethylformamide, at temperatures -30°C to 100°C, whereupon the solvent is evaporated and the protecting group on the heterocyclic base is removed by methanolysis or acid hydrolysis and, after isolation or deionisation at room temperature, the compounds of formula IV, as defined, wherein B signifies the same as in the formula I and R is the same as in the formula III, are reacted with bromotrimethylsilane in an aprotic solvent, preferably in acetonitrile, and after removal of the solvent the reaction mixture is treated with water or buffer solutions and the compounds of the general formula I are isolated, preferably by ion-exchange chromatography.

When B is hypoxanthin-9-yl, the method preferably comprises in reaction of compound of the formula I, where B is adenin-9-yl, with 3-methylbutyl nitrite in 50 - 80% aqueous acetic acid or with an aqueous solution of sodium nitrite in the presence of an equimolecular amount of acetic acid at room temperature.

When B is xanthin-9-yl, the method preferably comprises in reaction of a compound of formula I, where B is guanin-9-yl, with 3-methylbutyl nitrite in 50 - 80% aqueous acetic acid or with an aqueous solution of sodium nitrite in the presence of an equimolecular amount of acetic acid at room temperature.

When B is 2,6-diaminoadenin-9-yl, the method preferably comprises in reaction of a compound of formula I, where B is 2-amino-6-azidopurin-9-yl, with gaseous hydrogen in the presence of a catalyst, preferably metallic palladium, prereduced on an inorganic carrier, in water or in dilute aqueous solutions of organic or mineral acids.

The effect of compounds of the formula I is shown in Table 1.

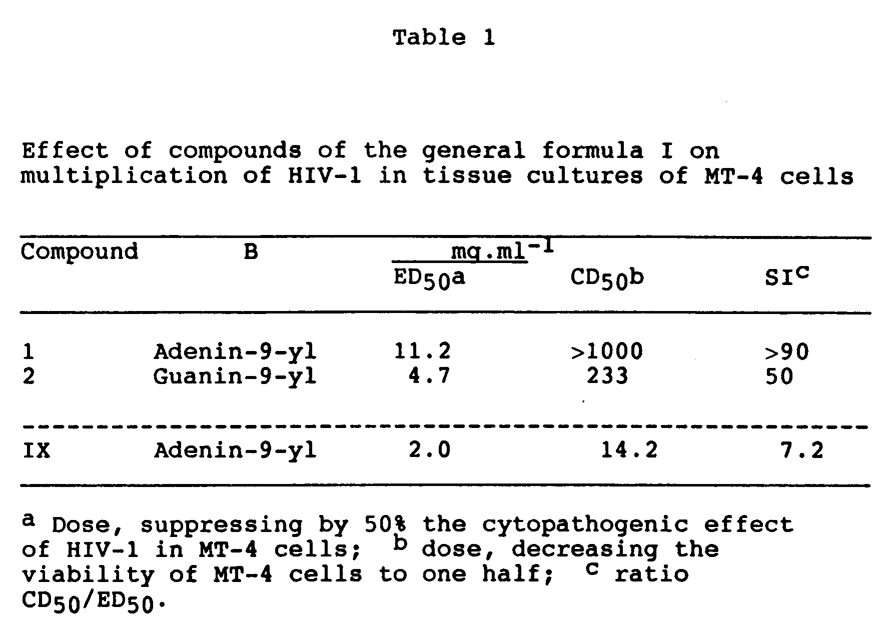

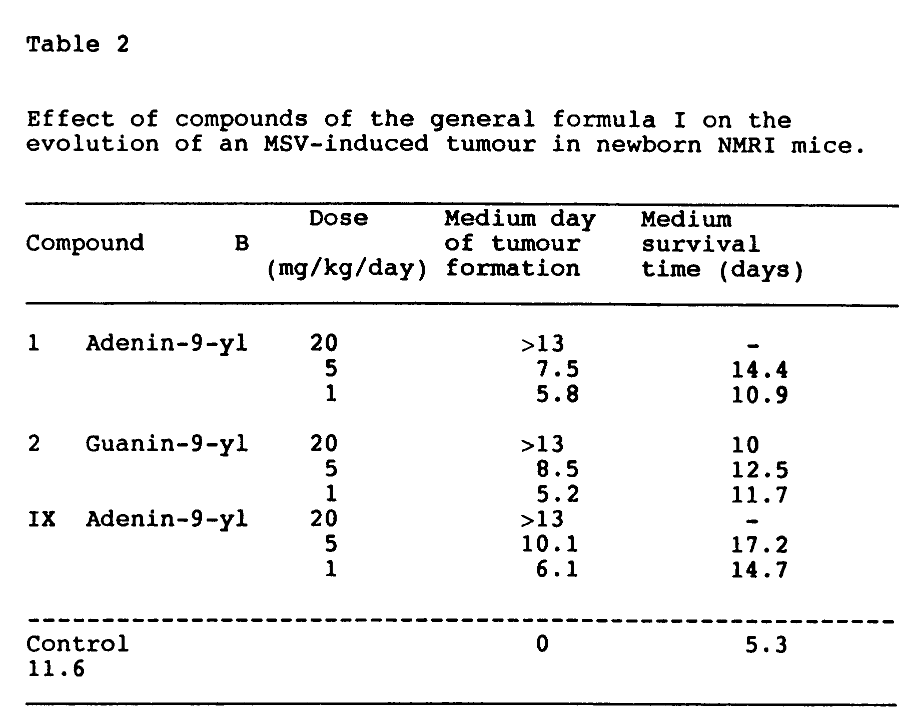

Methods of preparing compounds of the general formula I according to this invention are illustrated by the following Examples. Antiviral activity of the prepared compounds is given in Tables 1 and 2.

### Example 1

Chlorotrimethylsilane (24 ml) is added to a suspension of 9-(RS)-(3-fluoro-2-hydroxypropyl)adenine (6g) in pyridine (160 ml) and the mixture is stirred at room temperature for 1 hour. Benzoyl chloride (18.5 ml) is added and stirring at room temperature is continued for 2 hours. After cooling with ice, ice-cold water (30 ml) followed by concentrated aqueous ammonia (66 ml) is added and the mixture is stirred for 30 minutes under ice-cooling. The solid is collected, washed successively with water, acetone and ether and dried over phosphorus pentoxide at 15 Pa.

Yield 7.5g (84%) of 9-(RS)-(3-fluoro-2-hydroxypropyl)-N⁶-benzoyladenine, m.p. 197°C, R_{F} = 0.35 (TLC on Silufol UV 254 in chloroform-methanol 9:1). For C₁₅H₁₄FN₅O₃ (331.3) calculated: 54.38% C, 4.26% H, 5.73% F, 21.14% N; found: 54.62% Cg 4.46% H, 6.04% F, 23.67% N. ¹³C NMR spectrum: 45.91 d, CH₂N, ³J_{CF} = 8.0; 67.51 d, CH(OH), ²J_{CF} = 19.2; 85.08 d, CH₂F, ¹J_{CF} = 169.1.

The obtained product is codistilled with dimethylformamide (2 x 25 ml) at 4O°C/15 Pa, and dissolved in dimethylformamide (80 ml).

The solution is cooled to O°C, a 60% dispersion of sodium hydride in paraffin oil (2.8g) added, and the mixture stirred for 30 minutes. A solution of di(2-propyl) p-toluenesulphonyloxymethylphosphonate (9.1g) in dimethylformamide (20 ml) is added and stirring continued for 16hrs at 80°C under exclusion of moisture.

After evaporation of the solvent at 40°C/15 Pa, the residue is codistilled under the same conditions with toluene (2 x 25 ml) and then 0.1 M methanolic sodium methoxide (200 ml) is added. The mixture is allowed to stand at room temperature for 20hrs, neutralised by addition of a cation-exchanging resin in the H⁺-form (e.g. Dowex 50), made alkaline by addition of triethylamine and filtered. The solid on filter is washed with methanol (200 ml) and the combined filtrates are taken down in vacuo. The residue is applied onto a column of the same ion-exchanger (200 ml) which is then washed with 20% aqueous methanol. When the UV absorption (at 254 nm) of the eluate dropped to the original value, the product is eluted from the column with dilute (1:10 final concentration 2 - 2.5% wt/wt) aqueous ammonia solution (1 litre). The eluate is taken down in vacuo and the residue is codistilled with ethanol (2 x 50 ml), dried over phosphorus pentoxide at 15 Pa for 24 hours and mixed with acetonitrile (150 ml) and bromotrimethylsilane (15 ml). After standing for 24 hours at room temperature, the solvent is evaporated in vacuo, the residue is mixed with water (100 ml), set aside for 30 minutes and neutralized with concentrated aqueous ammonia. After evaporation in vacuo the residue is dissolved in water (50 ml), applied on a column of a cation-exchanger, (e.g. Dowex 50) in the H⁺-form (200 ml) and the above-described deionisation process is repeated. The ammonia eluate is evaporated and the residue, dissolved in water (50 ml) is applied onto a column of a medium basic anion-exchanger, (e.g. Dowex 1X2) (200 ml). The column is washed with water (400 ml) and then successively with 0.5 M acetic acid (1 litre) and 1 M acetic acid, the elution course being monitored by measurement of the UV absorption of the eluate at 254nm. The product is obtained from the 1 M acetic acid eluate from which the acetic acid is removed by evaporation in vacuo and by codistillation of the residue with water. The product is dissolved in boiling water (100 ml), the solution is filtered while hot and concentrated in vacuo to about 20 ml. Ethanol (100 ml) and ether (100 ml) are added and, after standing for 24hrs in a refrigerator, the product is collected, washed with ether and dried in vacuo. Yield 2.5g of 9-(RS)-(3-fluoro-2- phosphonylmethoxypropyl)-adenine, m.p. 265°C. For C₉H₁₃FN₅0₄P (305.3) calculated: 35.40% C, 4.29% H, 6.22% F, 22.95% N, 10.17% P; found: 35.69% C, 4.57% H, 7.25% F, 22.84% N, 9.71% P. ¹³C NMR spectrum: 43.12 d, CH₂N, ³J_{CF} = 6.8; 77.47 dd, CH, ²J_{CF} = 19.0, ³J_{PC} = 11.0; 82.32 d, CH₂F, ¹J_{CF} = 167.3; 68.37 d, CH₂P, ¹J_{PC} = 149.8. Electrophoretical mobility (relative to uridine 3′-phosphate) at pH 7.5: 0.88.

### Example 2

Chlorotrimethylsilane (27 ml) is added to a suspension of 1-(RS)-(3-fluoro-2-hydroxypropyl)cytosine (5.9g) in pyridine (180 ml) and the reaction mixture is further processed as described in Example 1. Yield 10g of 1-(RS)-(3-fluoro-2-hydroxypropyl)-N⁴-benzoylcytosine, m.p. 205°C. R_{F} = 0.42 (TLC, Silufol UV 254, chloroform methanol 9:1). For C₁₄H₁₄FN₃0₂ (291.3) calculated: 57.72% C, 4.84% H, 6.52% F, 14.43% N; found: 58.29% C, 4.63% H, 6.32% F, 14.19% N. ¹³C NMR spectrum: 45.91 d, CH₂N, ³J_{CF} = 8.0; 67,51 d, CH(OH), ²J_{CF} = 119.2; 85.08 d, CH₂F, ¹J_{CF} = 169.1.

This product is codistilled with dimethylformamide (2 x 25 ml) at 40°C/15 Pa, dissolved in dimethylformamide (100 ml), cooled to 0°C and mixed with 60% dispersion (3.8g) of sodium hydride in paraffin oil. After stirring for 30 minutes, a solution of di(2-propyl) p-toluene-sulphonyloxymethylphosphonate (11.6g) in dimethylformamide (20 ml) is added and the mixture is stirred under exclusion of moisture for 16 hours at 80°C. Further work-up procedure, the reaction of the deionized intermediate with bromotrimethylsilane and the subsequent processing and deionisation are performed as described in Example 1. The ammonia eluate is evaporated the residue is dissolved in water (50 ml) and applied onto a column of a medium basic anion exchanger, (e.g. Dowex 1X2; 200 ml). The column is washed with water (400 ml) and then the product is eluted with 0.5 M acetic acid; its elution is monitored by UV absorption measurement (at 254 nm) of the eluate. The UV absorbing fractions are evaporated in vacuo and the residue is freed from acetic acid by codistillation with water and purified by preparative liquid chromatography on a column of C18-silica gel (300 ml) in water. The product is dissolved in boiling water (100 ml), the hot solution is filtered and concentrated in vacuo to about 20 ml. Ethanol (100 ml) and ether (100 ml) are added, the mixture is set aside in a refrigerator for 24 hours, filtered and the product washed with other and dried in vacuo. Yield 2.0g of 1-(RS)-(3-fluoro-2- phosphonylmethoxypropyl)-cytosine, m.p. 219°C. For C₈H₁₃FN₃O₅P (281.3) calculated: 34.16% C, 4.66% H, 6.76% F, 14.94% N, 11.04% P; found: 34.63% C, 5.00% H, 6.70% F, 15.47% N, 11.15% P. ¹³C NMR spectrum: 49.42 d, CH₂N, ³J_{CF} = 7.1; 78.04 dd, CH, ²J_{CF} = 18.5, ³J_{PC} = 11.4; 82.78 d, CH₂F, ¹J_{CF} = 167.2; 67.70 d, CH₂P, ¹J_{PC} = 153.9. Electrophoretical mobility (relative to uridine 3′-phosphate) at pH 7.5: 0.95.

### Example 3

Chlorotrimethylsilane (17 ml) is added to a suspension of 9-(RS)-(3-fluoro-2-hydroxypropyl)guanine (4.7g) in pyridine (120 ml) and, after stirring for 1 hour, benzoyl chloride (13 ml) is added. After 2 hours the mixture is decomposed at 0°C with water (20 ml) and concentrated aqueous ammonia (45 ml) and worked up as described in Example 1. Yield 7.2g of 9-(RS)-(3-fluoro-2-hydroxypropyl)-N²-benzoylguanine, m.p. 178°C; R_{F} = 0.20 (TLC, Silufol UV 254, chloroform - methanol 9:1). For C₁₅H₁₄FN₅0₃ (331.3) calculated: 54.38% C, 4.26% H, 5.73% F, 21.14% N; found: 54.82% C, 5.07% H, 5.71% F, 21.29% N. ¹³C NMR spectrum: 45.73 s, CH₂N, ³J_{CF} = 7.1; 67.54 d, CH(OH), ²J_{CF} = 19.5; 85.04 d, CH₂F, ¹J_{CF} = 168.5.

This product is codistilled with dimethylformamide (2 x 25 ml) at 40°C/15 Pa, dissolved in dimethylformamide (80 ml), cooled to -20°C and mixed with 60% dispersion of sodium hydride in paraffin oil (2.4g) and with a solution of di(2-propyl) p-toluenesulphonyloxymethylphosphonate (7.7g) in dimethylformamide (20 ml). The mixture is stirred at 70°C for 16 hours under exclusion of moisture. Further work-up of the mixture, reaction of the deionized intermediate with bromotrimethylsilane and the subsequent work-up and deionisation are executed as described in Example 1. The ammonia eluate is evaporated and the residue is dissolved in water (50 ml) and applied onto a column of medium basic anion-exchanger (120 ml; e.g. Dowex 1X2). The column is washed with water (400 ml) and then the product is eluted with 0.5 M acetic acid, the course of the elution being followed by measurement of the UV absorption of the eluate at 254 nm. The UV-absorbing eluate is taken down in vacuo and the acetic acid is removed by codistillation of the residue with water. The product is crystallized from water; yield 2.3g of 9-(RS)-3-fluoro-2-phosphonylmethoxypropyl)guanine, not melting up to 300°C. For C₉H₁₃FN₅0₅P (321.1) calculated: 33.64% C, 4.08% H, 5.91% F, 21.80% N, 9.66% P; found: 34.00% C, 3.75% H, 5.60% F, 21.87% N, 10.20% P. ¹³C NMR spectrum: 42.22 d, CH₂N, ³J_{CF} = 8.3; 77.52 dd, CH, ²J_{CF} = 19.0, ³J_{PC} = 11.3; 82-05 d, CH₂F, ¹J_{CF} = 166.8; 67.96 d, CH₂P, ¹J_{PC} = 150.1. Electrophoretical mobility (relative to uridine 3′-phosphate)at pH 7.5: 0.92.

### Example 4

Chlorotrimethylsilane (8.5 ml) is added to a suspension of 9-(RS)-3-fluoro-2-hydroxypropyl)-2 -amino-6-azidopurine (4.7g) in pyridine (60 ml), the mixture is stirred for 1hour and then benzoyl chloride (6.5ml) is added. After 2 hours the mixture is decomposed at 0°C with water (10 ml) and concentrated aqueous ammonia (24 ml). The mixture is worked up as described in Example 1 to give 3.6g of 9-(RS)-(3-fluoro-2-hydroxypropyl)-2-benzoylamino-6-azidopurine, m.p. 198°C, R_{F} = 0.30 (TLC on Silufol UV 254, chloroform-methanol 9:1). For C₁₅H₁₃FN₈0₂ (356.3) calculated: 50.56% C, 3.68% H, 5.33% F, 31.45% N; found: 50.42% C, 4.08% H, 5.32% F, 31.25% N.

This product is codistilled with dimethylformamide (2 x 25ml) at 40°C/15 Pa, dissolved in dimethylformamide (40 ml), cooled to -20°C and mixed with 60% dispersion of sodium hydride in paraffin oil (1.1g) and di(2-propyl) p-toluenesulphonyloxymethylphosphonate (3.8g) in dimethylformamide (10 ml). The mixture is stirred at room temperature for 16 hours under exclusion of moisture. Further work-up of the mixture, reaction of the deionized intermediate with bromotrimethylsilane (80 ml of acetonitrile and 8 ml of bromotrimethylsilane), the subsequent work-up and deionisation are performed as described in Example 1. After washing out the salts with water from the cation-exchanger column, the product is eluted with a considerable retention with water. The product-containing fractions are taken down in vacuo and the residue is crystallized from water to give 2.4g of 9-(RS)-(3-fluoro-2-phosphonylmethoxypropyl)-2-amino-6-azidopurine, not melting up to 260°C. For C₉H₁₂FN₈0₄P (346.3) calculated: 31.21% C, 3.49% H, 5.49% F, 32.36% N, 8.96% P; found: 31.56% C, 3.70% H, 5.62% F, 32.56% N, 9.21% P. Electrophoretical mobility (relative to uridine 3′-phosphate) at pH 7.5: 0.92.

### Example 5

Chlorotrimethylsilane (7 ml) is added to a suspension of 9-(RS)-3-fluoro-2-phosphonylmethoxypropyl)-3-deazaadenine (9 mmol) in pyridine (50 ml). After 1 hour benzoyl chloride (5.5 ml) is added. After a further 2 hours the mixture is decomposed at 0°C by gradual addition of water (7 ml) and concentrated aqueous ammonia (18 ml) and after 30 minutes the solvents are evaporated in vacuo at 0°C. The residue is codistilled in vacuo and chromatographed on a column of silica gel (200 ml) in chloroform. The product is eluted with chloroform -methanol (95:5). Evaporation of the solvents in vacuo afforded 1.60g of 9-(RS)-(3-fluoro-2-phosphonylmethoxypropyl)-N⁶ -benzoyl-3-deazaadenine as an amorphous foam, R_{F} = 0.12 (TLC on Silufol UV 254, chloroform - methanol 9:1).

This product is mixed with a solution of di(2-propyl) p-toluenesulphonyloxymethylphosphonate (2g) in dimethylformamide (10 ml) and, after cooling to -15°C, 60% dispersion of sodium hydride in paraffin oil (600 mg) is added. The reaction mixture is stirred for 16 hours at room temperature under exclusion of moisture. Further work-up of the mixture, reaction of the deionized intermediate with bromotrimethylsilane (50ml of acetonitrile and 5 ml of bromotrimethylsilane), the subsequent work-up and deionisation are performed as described in Example 1. The column of the cation-exchanger is eluted with water. After washing out the salts, the product is eluted with dilute aqueous ammonia. The product-containing fractions are evaporated in vacuo and the residue is purified by preparative chromatography on C18-silica gel (elution with water). Yield 0.50g of 9-(RS)-(3-fluoro-2-phosphonylmethoxypropyl)-3-deazaadenine, not melting up to 260°C. For C₁₀H₁₄FN₄0₄P (304.3) calculated: 39.47% C, 4.64% H, 6.24% F, 18.42% N, 10.20% P; found: 39.80% C, 4.67% H, 6.38% F, 18.67% N, 10.50% P. Electrophoretical mobility (relative to uridine 3'-phosphate) at pH 7.5: 0.78.

### Example 6

3-Methylbutyl nitrite (2 ml) is added to a suspension of 9-(RS)-(3-fluoro-2-phosphonylmethoxy propyl)adenine (200 mg) in 80% aqueous acetic acid (20 ml) and the mixture is allowed to stand at room temperature for 24 hours. After evaporation in vacuo, the residue is codistilled with water (4 x 20 ml) and applied on a column of a cation-exchanger in H⁺-form (150 ml, e.g. Dowex 50). The column is washed with water and the elution is followed by measurement of UV absorption of the eluate at 254 nm. The UV-absorbing fractions are combined and evaporated in vacuo and the residue is codistilled with ethanol (2 x 25 ml), mixed with ether and collected on filter. Yield 100 mg of 9-(RS)-(3-fluoro-2-phosphonylmethoxypropyl)hypoxanthine, not melting up to 260°C. For C₉H₁₂FN₄0₅P (306.3) calculated: 35.29% C, 3.95% H, 6.20% F, 18.30% N, 10.13% P; found: 35.15% C, 4.12% H, 6.43% F, 18.70% N, 9.87% P. Electrophoretical mobility (relative to uridine 3'-phosphate) at pH 7.5: 0,83.

### Example 7

A suspension of 9-(RS)-(3-fluoro-2-phosphonylmethyloxypropyl)guanine in a mixture of 80% aqueous acetic and (20 ml) and 3-methylbutyl nitrite (2 ml) is allowed to stand at room temperature for 24 hours and processed further as described in Example 5. Yield 100mg of 9-(RS)-(3-fluoro-2-phosphonylmethoxy propyl)xanthine, not melting up to 260°C. For C₉H₁₂FN₄O₆P (322.3) calculated: 33.54% C, 3.75% H, 5.90% F, 17.39% N, 9.63% P; found: 33.65% C, 3.90% H, 5.77% F, 17.56% N, 9.40% P. Electrophoretical mobility (relative to uridine 3′-phosphate) at pH 7.5: 0.78.

### Example 8

To a suspension of 9-(RS)-(3-fluoro-2-phosphonylmethoxpropyl)-2-amino-6-azidopurine (200mg) in 80% aqueous acetic acid (50 ml) is added 10% palladium on active carbon (200 mg) and the mixture is stirred in an atmosphere of hydrogen at atmospheric pressure for 24 hours. The reaction mixture is filtered, the catalyst washed with water and the filtrate codistilled with water (3 x 20 ml). The crude product is purified by chromatography on a column of C18 silica gel in water and, after evaporation, the product is dissolved in hot water, mixed with a fivefold volume of ethanol, and ether is added to incipient turbidity. After standing in a refrigerator, the product is collected on filter, washed with ether and dried in vacuo, Yield 100 mg of 9-(RS)-(3-fluoro-2-phosphonylmethoxypropyl)-2,6-diaminopurine, not melting up to 260°C. For C₉H₁₄FN₆O₄P (320.3) calculated: 33.75% C, 4.41% H, 5.93% F, 26.24% N, 9.69% P; found: 33.65% C, 3.95% H, 5.79% F, 26.56% N, 9.42% P. Electrophoretical mobility (relative to uridine 3′-phosphate) at PH 7.5: 0.70.

### Preparative Example.

### Di(2-propyl) p-toluenesulphonyloxymethylphosphonate

Triethylamine (8.4 ml) is added to a stirred mixture of di(2-propyl)phosphite (96g) and paraformaldehyde (23g), and the mixture is heated to 100°C (reflux condenser, calcium chloride protecting group) until the paraformaldehyde dissolves completely. After heating for one more hour, the reaction mixture is cooled and mixed with acetonitrile (500 ml) and p-toluenesulphonyl chloride (121.5g). Triethylamine (90 ml) and 4-dimethylaminopyridine are then added under stirring. After stirring for 24hrs the mixture is cooled with ice, 0.4 M triethylammonium hydrogen carbonate (400 ml) is gradually added and the mixture is again stirred for 24hrs. Acetonitrile is evaporated in vacuo (bath temperature below 40°C) and the mixture is extracted with ether (3 x 200 ml). The ethereal extract is dried over sodium sulphate, filtered, and the solvent is evaporated in vacuo. The residue is dissolved in benzene (200 ml) and filtered through a column of silica gel (500 ml, prepared in benzene). The column is washed with benzene until impurities of higher R_{F} are removed (monitoring by TLC on silica gel plates in chloroform) and then the product is eluted with ethyl acetate. After evaporation of the solvent in vacuo, the remaining oil is stirred with light petroleum (300 ml) to crystallisation, the crystals are collected, washed with light petroleum and dried in vacuo. Yield 140 g of di(2-propyl) p-toluenesulphonyl-oxymethylphosphonate, m.p. 37.5°C. For C₁₄H₂₃PO₆S (350.4) calculated: 47.98% C, 6.61% H, 8.86% P, 9.15% S; found: 48.15% C, 6.80% H, 9.02% P; 9.24% S.

## Claims

1. A compound of formula (I): 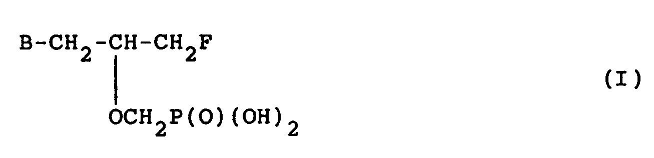 wherein B represents a purin-9-yl or pyrimidin-1-yl moiety, and pharmaceutically acceptable derivatives thereof, especially the alkali metal salts, ammonia or amine salts.

2. A compound according to claim 1, wherein the purine or pyrimidine component is substituted with one or more substituents selected from: amino groups, hydroxy groups, keto groups, cyano groups, alkylamino groups, halogen atoms, thiol groups, lower alkyl groups, either unsubstituted or further substituted with similar groups as defined above, and alkenyl or alkynyl groups, unsubstituted or substituted as defined for alkyl groups.

3. A compound according to any preceding claim, wherein the purine or pyrimidine component is hypoxanthin-9-yl, xanthin-9-yl, or 2,6-diaminoadenin-9-yl.

4. A compound according to any preceding claim, wherein the base is a pyrimidine component of formula (V): 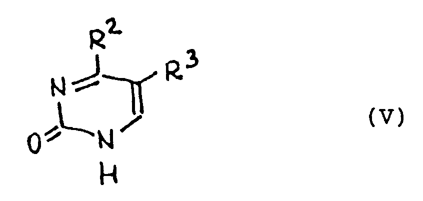 wherein R² is a hydrogen atom, a hydroxylamino, hydrazino, alkoxy, amino or substituted amino group and R³ is a hydrogen or halogen atom or an alkyl (C₁-C₃) group, or moieties of the general formula VI 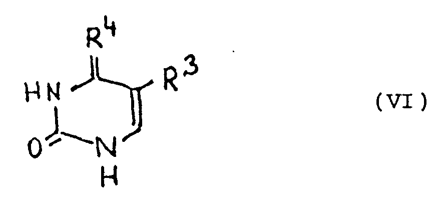 wherein R³ is as in formula V and R⁴ is an atom of oxygen or sulphur, as well as their synthetic analogues such as aza (5-aza, 6-aza) or deaza (3-deaza) derivatives.

5. A compound according to any one of claims 1 to 3, wherein the base is a purine component of formula VII 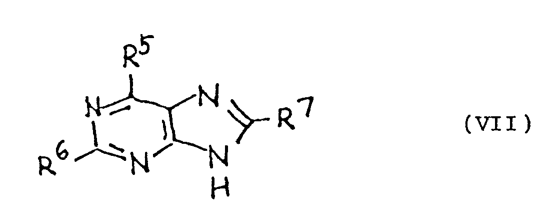 where R⁵ and R⁶ are a hydrogen or halogen atom, an amino, hydrazino, hydroxylamino, azido, substituted amino, hydroxy, alkoxy, mercapto, alkylmercapto group, or an alkyl group with a short carbon chain (C₁-C₄), and combination of these groups, and R₇ is an atom of hydrogen or halogen, a hydroxy, alkoxy, mercapto, alkylthio, amino or substituted amino group, and synthetic analogues, such as aza (2-aza, 8-aza) or deaza (1-deaza, 3-deaza or 7-deaza) derivatives.

6. Use of a compound according to any of claims 1 to 5 in the manufacture of a medicament for the treatment or prophylaxis of a retroviral condition.

7. A method for the preparation of a compound according to any preceding claim, wherein a phosphonomethyl group is introduced to the free hydroxy group of a N-(3-fluoro-2-hydroxypropyl) derivative of a heterocyclic base of formula II 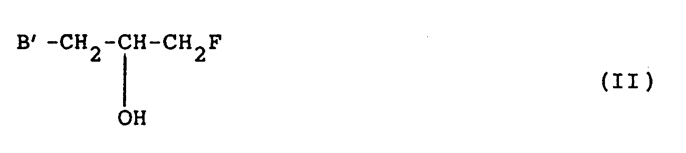 where B' is a purin-9-yl or pyrimidin-1-yl moiety or a protected derivative thereof.

8. A method according to claim 7, wherein the protecting groups in the protected derivative are O-alkyl, N-acyl or N-dialkylaminomethylene groups.

9. A method according to claim 7 or claim 8, wherein the compound of formula II is reacted with a compound of formula III
4-CH₃C₆H₄SO₂OCH₂P(O)(OR¹)₂ (III)
wherein R¹ is a lower alkyl , in the presence of 1 - 5 molar equivalents (relative to compounds of the general formula II) of sodium hydride in a dipolar aprotic solvent, at temperatures of -30°C to 1OO°C, whereupon the solvent is removed and the protecting group on the heterocyclic base is removed, and, after isolation or deionisation, the compounds of formula IV 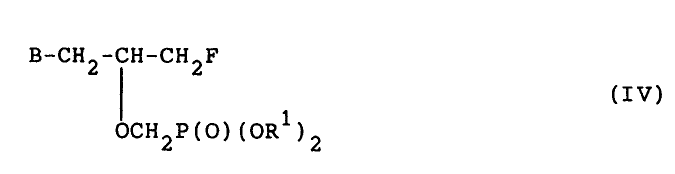 wherein B signifies the same as in the formula I and R¹ is the same as in formula III, are reacted with halotrimethylsilane in an aprotic solvent, and the solvent is removed.

10. A method according to claim 9, wherein R¹ is a C₁-C₃ alkyl group.

11. A method according to claim 9, wherein R¹ is a 2-propyl group

12. A method according to claim 9, wherein the dipolar aprotic solvent is dimethylformamide

13. A method according to claim 9, wherein the protecting group on the heterocyclic base is removed by methanolysis or acid hydrolysis,

14. A method according to claim 9, wherein the halotrimethylsilane is bromotrimethylsilane.

15. A method according to claim 9, wherein the aprotic solvent is acetonitrile.

16. A method according to claim 9, wherein, after removal of the solvent, the reaction mixture is optionally treated with water or buffer solutions and the desired compounds isolated.

17. A method according to claim 16, wherein the desired compounds are isolated by ion-exchange chromatography.

## Patentansprüche

1. Verbindung der Formel (I): 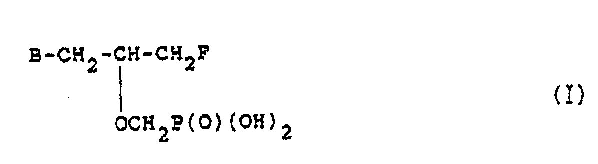 wobei B ein Purin-9-yl- oder Pyrimidin-1-yl-Anteil ist, und pharmazeutisch annehmbare Derivate davon, insbesondere Alkalimetallsalze. Ammoniak- oder Aminsalze.

2. Verbindung nach Anspruch 1, wobei die Purin- oder Pyrimidinkomponente durch einen oder mehrere Substituenten ersetzt wird, die unter den folgenden ausgewählt sind: Aminogruppen, Hydroxygruppen, Ketogruppen, Cyanogruppen, Alkylaminogruppen, Halogenatome, Thiolgruppen, niedere Alkylgruppen, entweder nichtsubstituiert oder weiter mit ähnlichen Gruppen wie den oben definierten substituiert, und Alkenyl- oder Alkinylgruppen, nichtsubstituiert oder gemäß der obigen Definition für Alkylgruppen substituiert.

3. Verbindung nach einem der vorhergehenden Ansprüche, wobei die Purin- oder Pyrimidinkomponente Hypoxanthin-9-yl, Xanthin-9-yl oder 2,6-Diaminoadenin-9-yl ist.

4. Verbindung nach einem der vorhergehenden Ansprüche, wobei die Base eine Pyrimidinkomponente mit dem Formel (V): 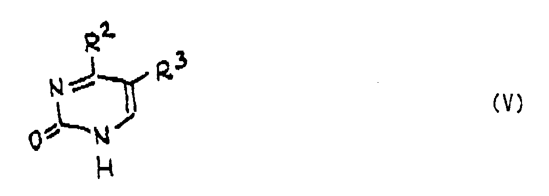 wobei R² ein Wasserstoffatom, eine Hydroxylamino-, Hydrazino-, Alkoxy-, Amino- oder substituierte Aminogruppe bedeuten und R³ ein Wasserstoff- oder Halogenatom oder eine (C₁-C₃)-Alkylgruppe bedeutet, oder Anteile mit der allgemeinen Formel VI, 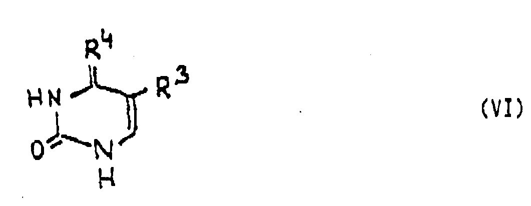 wobei R³ die gleiche Bedeutung wie in Formel V hat und R⁴ ein Sauerstoff- oder Schwefelatom ist, sowie deren synthetische Analoge darstellt, wie z. B. Aza- (5-Aza-, 6-Aza-) oder Desaza- (3-Desaza-) Derivate.

5. Verbindung nach einem der Ansprüche 1 bis 3, wobei die Base eine Purinkomponente mit der Formel VII, 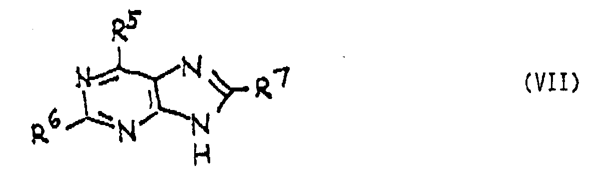 wobei R⁵ und R⁶ ein Wasserstoff- oder Halogenatom, eine Amino-, Hydrazino-, Hydroxylamino, Azido-, substituierte Amino-, Hydroxy-, Alkoxy-, Mercapto-, Alkylmercaptogruppe, oder eine Alkylgruppe mit kurzer Kohlenstoffkette (C₁-C₄), sowie eine Kombination aus diesen Gruppen bedeuten, und R⁷ ein Wasserstoff- oder Halogenatom, eine Hydroxy-, Alkoxy-, Mercapto-, Alkylthio-, Amino- oder substituierte Aminogruppe bedeutet, sowie synthetische Analoge darstellt, wie z. B. Aza- (2-Aza-, 8-Aza-) oder Desaza- (1-Desaza-, 3-Desaza- oder 7-Desaza-) Derivate.

6. Verwendung einer Verbindung nach einem der Ansprüche 1 bis 5 bei der Herstellung eines Medikaments zur Behandlung oder Prophylaxe eines retroviralen Zustands.

7. Verfahren zur Herstellung einer Verbindung nach einem der vorhergehenden Ansprüche, wobei eine Phosphonmethylgruppe an die freie Hydroxygruppe eines N-(3-Fluor-2-hydroxypropyl)-Derivats einer heterocyclischen Base mit der Formel II gebunden wird, 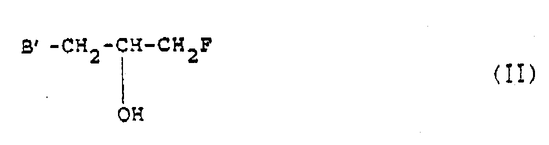 wobei B' ein Purin-9-yl- oder Pyrimidin-1-yl-Anteil oder ein geschütztes Derivat dieser Komponenten ist.

8. Verfahren nach Anspruch 7, wobei die Schutzgruppen in dem geschützten Derivat O-Alkyl-, N-Acyl- oder N-Dialkylaminomethylen-Gruppen sind.

9. Verfahren nach Anspruch 7 oder Anspruch 8, wobei die Verbindung mit Formel II mit einer Verbindung der Formel III.
4-CH₃C₆H₄SO₂OCH₂P(O)(OR¹)₂ (III)
wobei R¹ ein niederen Alkylrest ist, in Gegenwart von 1-5 Moläquivalenten (bezogen auf die Verbindungen mit der allgemeinen Formel II) Natriumhydrid in einem dipolarer aprotischen Lösungsmittel bei Temperaturen von -30°C bis 100°C umgesetzt wird, worauf das Lösungsmittel entfernt wird und die Schutzgruppe an der heterocyclischen Base entfernt wird und, nach Isolierung oder Deionisation, die Verbindungen der Formel IV, 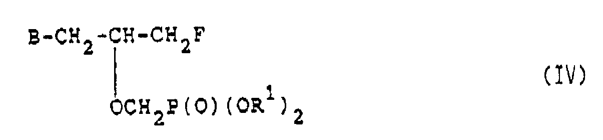 wobei B die gleiche Bedeutung wie in Formel I und R¹ die gleiche Bedeutung wie in Formel III haben, in einem aprotischen Lösungsmittel mit Halogentrimethylsilan zur Reaktion gebracht werden und das Lösungsmittel entfernt wird.

10. Verfahren nach Anspruch 9, wobei R¹ eine C₁-C₃-Alkylgruppe ist.

11. Verfahren nach Anspruch 9, wobei R¹ eine 2-Propylgruppe ist.

12. Verfahren nach Anspruch 9, wobei das dipolare aprotische Lösungsmittel Dimethylformamid ist.

13. Verfahren nach Anspruch 9, wobei die Schutzgruppe an der heterocyclischen Base durch Methanolyse oder Säurehydrolyse entfernt wird.

14. Verfahren nach Anspruch 9, wobei das Halogentrimethylsilan Bromtrimethylsilan ist.

15. Verfahren nach Anspruch 9, wobei das aprotische Lösungsmittel Acetonitril ist.

16. Verfahren nach Anspruch 9, wobei, nach Entfernen des Lösungsmittels, das Reaktionsgemisch wahlweise mit Wasser oder Pufferlösungen behandelt wird und die gewünschten Verbindungen isoliert werden.

17. Verfahren nach Anspruch 16, wobei die gewünschten Verbindungen durch Ionenaustauschchromatographie isoliert werden.

## Revendications

1. Composé de formule (I): 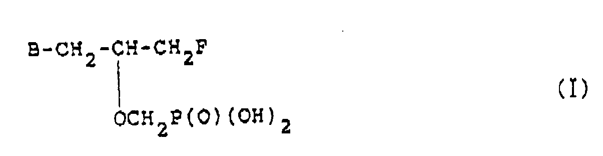 où B représente une fraction purine-9-yle ou pyrimidine-1-yle, et des dérivés de celui-ci pharmaceutiquement acceptables, en particulier les sels de métal alcalin, les sels d'ammoniac ou d'amines.

2. Composé selon la revendication 1, dans lequel le constituant purine ou pyrimidine est substitué par un ou plusieurs substituants choisis parmi: les groupes aminos, les groupes hydroxyles, les groupes cétoniques, les groupes cyanos, les groupes alkylaminos, les atomes d'halogène, les groupes thiols, les groupes alkyles inférieurs, soit non substitués soit substitués par des groupes semblables à ceux définis ci-dessus, et les groupes alcényles ou alcynyles, non substitués ou substitués comme défini pour les groupes alkyles.

3. Composé selon l'une quelconque des revendications précédentes, dans lequel le constituant purine ou pyrimidine est l'hypoxanthine-9-yle, le xanthine-9-yle ou le 2,6-diaminoadénine-9-yle.

4. Composé selon l'une quelconque des revendications précédentes, dans lequel la base est un constituant pyrimidine de formule (V): 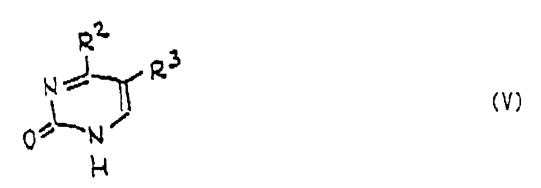 où R² est un atome d'hydrogène, un groupe hydroxylamino, hydrazino, alcoxy. amino ou amino substitué, et R³ est un atome d'hydrogène ou d'halogène ou un groupe alkyle en C₁ à C₃, ou des fractions de formule générale VI 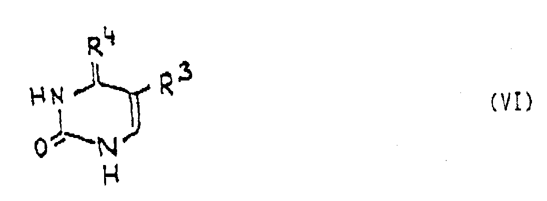 où R³ est tel que dans la formule V et R⁴ est un atome d'oxygène ou de soufre, ainsi que leurs analogues synthétiques tels que les dérivés azas (5-aza, 6-aza) ou déazas (3-déaza).

5. Composé selon l'une quelconque des revendications 1 à 3, dans lequel la base est un constituant purine de formule VII 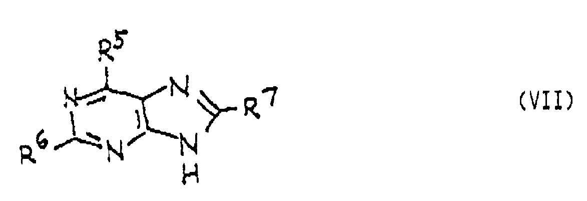 où R⁵ et R⁶ sont un atome d'hydrogène ou d'halogène, un groupe amino, hydrazino, hydroxylamino, azido, amino substitué, hydroxyle, alcoxy, mercapto, alkylmercapto, ou un groupe alkyle ayant une chaîne carbonée courte (C₁ à C₄), et une combinaison de ces groupes, et R⁷ est un atome d'hydrogène ou d'halogène, un groupe hydroxy, alcoxy, mercapto, alkylthio, amino ou amino substitué, et les analogues synthétiques tels que les dérivés azas (2-aza, 8-aza) ou déazas (1-déaza, 3-déaza ou 7-déaza),

6. Utilisation d'un composé selon l'une quelconque des revendications 1 à 5 dans la fabrication d'un médicament pour le traitement ou la prophylaxie d'un état rétroviral.

7. Procédé pour la préparation d'un composé selon l'une quelconque des revendications précédentes, dans lequel un groupe phosphonométhyle est lié au groupe hydroxyle libre d'un dérivé N-(3-fluoro-2-hydroxypropyle) d'une base hétérocyclique de formule II 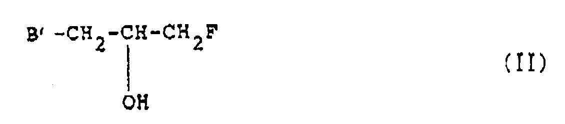 où B' est une fraction purine-9-yle ou pyrimidine-1-yle, ou un dérivé protégé de celle-ci.

8. Procédé selon la revendication 7, dans lequel les groupes protecteurs dans le dérivé protégé sont des groupes O-alkyles, N-acyles ou N-dialkylaminométhylènes.

9. Procédé selon la revendication 7 ou la revendication 8, dans lequel on fait réagir le composé de formule II avec un composé de formule III
4-CH₃C₆H₄SO₂OCH₂P(O)(OR¹)₂ (III)
où R¹ est un groupe alkyle inférieur, en présence de 1 à 5 équivalents molaires (par rapport aux composés de formule générale II) d'hydrure de sodium dans un solvant aprotique dipolaire, à des températures de -30°C à 100°C, après quoi on élimine le solvant et on élimine le groupe protecteur sur la base hétérocyclique, et, après isolement ou désionisation, on fait réagir les composés de formule IV 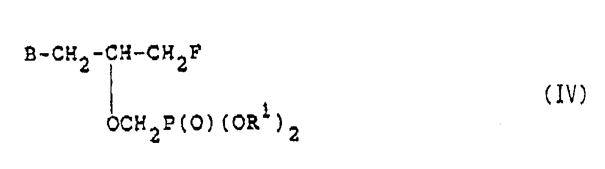 où B a la même signification que dans la formule I et R¹ est le même que dans la formule III, avec un halogénotriméthylsilane dans un solvant aprotique, puis on élimine le solvant.

10. Procédé selon la revendication 9, dans lequel R¹ est un groupe alkyle en C₁ à C₃.

11. Procédé selon la revendication 9, dans lequel R¹ est un groupe 2-propyle.

12. Procédé selon la revendication 9, dans lequel le solvant aprotique dipolaire est le diméthylformamide.

13. Procédé selon la revendication 9, dans lequel le groupe protecteur sur la base hétérocyclique est éliminé par méthanolyse ou hydrolyse acide.

14. Procédé selon la revendication 9, dans lequel l'halogénotriméthylsilane est le bromotriméthylsilane.

15. Procédé selon la revendication 9, dans lequel le solvant aprotique est l'acétonitrile.

16. Procédé selon la revendication 9, dans lequel, après l'élimination du solvant, le mélange réactionnel est éventuellement traité avec de l'eau ou des solutions tampons, puis les composés désirés sont isolés.

17. Procédé selon la revendication 16, dans lequel les composés désirés sont isolés par chromatographie d'échange ionique.
